(19) Europäisches Patentamt
European Patent Office
Office européen des brevets

(11) EP 0 894 791 A1

(12) **EUROPEAN PATENT APPLICATION**
published in accordance with Art. 158(3) EPC

(43) Date of publication:
**03.02.1999 Bulletin 1999/05**

(21) Application number: **97907441.6**

(22) Date of filing: **24.03.1997**

(51) Int. Cl.[6]: **C07D 239/38**, A01N 43/54

(86) International application number:
**PCT/JP97/00969**

(87) International publication number:
**WO 97/36878 (09.10.1997 Gazette 1997/43)**

(84) Designated Contracting States:
**DE ES FR GB IT**

(30) Priority: **29.03.1996 JP 76743/96**

(71) Applicant: **Ube Industries, Ltd.**
**Ube-shi, Yamaguchi-ken 755-0052 (JP)**

(72) Inventors:
- **NISHIMURA, Takashi, Ube Industries, Ltd. Ube-shi, Yamaguchi 755 (JP)**
- **TANAKA, Toshinobu, Ube Industries, Ltd. Ube-shi, Yamaguchi 755 (JP)**
- **YOKOYAMA, Shuji, Ube Industries, Ltd. Ube-shi, Yamaguchi 755 (JP)**
- **MORITOMO, Masaru, Ube Industries, Ltd. Ube-shi, Yamaguchi 755 (JP)**
- **HAYASHI, Keisuke, Ube Industries, Ltd. Ube-shi, Yamaguchi 755 (JP)**
- **MURAKAMI, Tadashi, Ube Industries, Ltd. Ube-shi, Yamaguchi 755 (JP)**

(74) Representative:
**Grosset-Fournier, Chantal Catherine et al**
**Grosset-Fournier & Demachy s.a.r.l.,**
**103 rue La Fayette**
**75481 Paris Cédex 10 (FR)**

(54) **PYRIMIDINE COMPOUNDS, PROCESS FOR PRODUCING THE SAME, AND AGRICULTURAL OR HORTICULTURAL BACTERICIDES**

(57) A pyrimidine compound represented by the following formula (1):

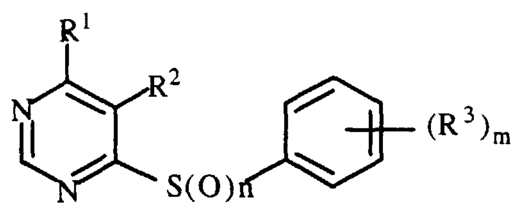

(1)

wherein $R^1$ represents a $C_{1-4}$ alkyl group, a halogen atom or a hydrogen atom; $R^2$ represents a halogen atom, a $C_{1-4}$ alkyl group or a hydrogen atom; $R^3$ represents a $C_{1-8}$ alkyl group, a $C_{1-4}$ haloalkyl group, a halogen atom, a nitro group, a $C_{1-4}$ alkoxy group, a benzoylamino group, a phenoxy group, a $C_{2-5}$ alkenyloxy group, a $C_{2-5}$ alkynyloxy group or a $C_{2-10}$ alkylcarbonylamino group; m represents an integer of 0 to 5; and n represents 1 or 2,

a process for preparing the same and an agricultural and horticultural fungicide containing the same as an effective ingredient.

EP 0 894 791 A1

## Description

Technical field

This invention relates to a novel pyrimidine compound, processes for preparing the same, and an agricultural and horticultural fungicide containing the same as an active ingredient.

Background art

Since the 4-substituted phenylsulfinylpyrimidine compound (or 4-substituted phenylsulfonylpyrimidine compound) of the present invention is a novel compound, it has not been known that the compound has an agricultural and horticultural fungicidal activity.
An object of the present invention is to provide a novel 4-substituted phenylsulfinylpyrimidine compound (or 4-substituted phenylsulfonylpyrimidine compound), processes for preparing the same, and an agricultural and horticultural fungicide containing the same as an active ingredient.

Disclosure of the invention

The present inventors have studied to solve the above-mentioned problems and as the results, they have found that the novel pyrimidine compound represented by the following formula (1) has remarkable agricultural and horticultural fungicidal activity whereby accomplished the present invention.
That is, the present invention is as mentioned below.
The first invention relates to a pyrimidine derivative represented by the following formula (1):

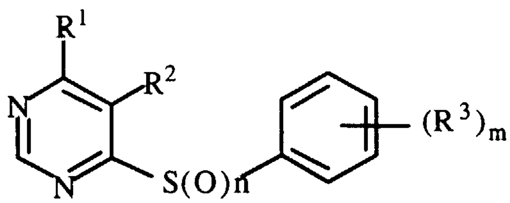

(1)

wherein $R^1$ represents an alkyl group having 1 to 4 carbon atoms, a halogen atom or a hydrogen atom; $R^2$ represents a halogen atom, an alkyl group having 1 to 4 carbon atoms or a hydrogen atom; $R^3$ represents an alkyl group having 1 to 8 carbon atoms, a haloalkyl group having 1 to 4 carbon atoms, a halogen atom, a nitro group, an alkoxy group having 1 to 4 carbon atoms, a substituted or unsubstituted benzoylamino group, a substituted or unsubstituted phenoxy group, an alkenyloxy group having 2 to 5 carbon atoms, an alkynyloxy group having 2 to 5 carbon atoms, a cycloalkylcarbonylamino group having 4 to 9 carbon atoms, a haloalkylcarbonyl amino group having 2 to 5 carbon atoms, a cyano group, a formyl group, an acyl group having 2 to 5 carbon atoms, a hydrogen atom or an alkylsulfonyl group having 1 to 4 carbon atoms; m represents an integer of 0 to 5; and n represents 1 or 2.

The second invention relates to a compound represented by the following formula (4):

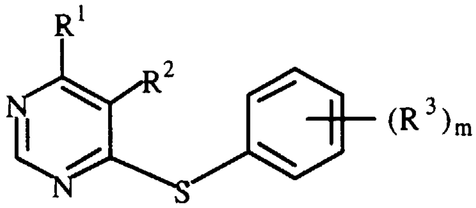

(4)

wherein $R^1$ to $R^3$ and m have the same meanings as defined above.

The third invention relates to a process for producing a pyrimidine compound represented by the formula (1) as mentioned above which comprises oxidizing the compound of the above-mentioned formula (4) obtained by reacting a compound represented by the following formula (2):

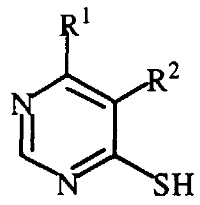

(2)

wherein $R^1$ and $R^2$ have the same meanings as defined above,

and a compound represented by the following formula (3):

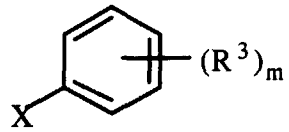

(3)

wherein $R^3$ has the same meaning as defined above. X represents a halogen atom.

The fourth invention relates to a process for preparing the pyrimidine compound represented by the formula (1) as mentioned above which comprises oxidizing the compound of the above-mentioned formula (4) obtained by reacting a compound represented by the following formula (5):

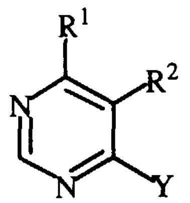

(5)

wherein $R^1$ and $R^2$ have the same meanings as defined above.
Y represents a halogen atom,

and a compound represented by the following formula (6):

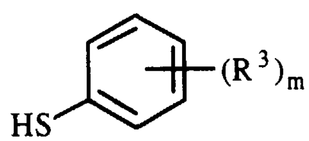

(6)

wherein $R^3$ and m have the same meanings as defined above.

The fifth invention relates to an agricultural and horticultural fungicide containing the pyrimidine compound represented by the formula (1) as mentioned above as an effective ingredient.

Best mode for practicing the invention

In the following, the pyrimidine compound which is a desired compound represented by the formula (1) is described as Compound (1), and the starting compounds represented by the formulae (2), (3), (4), (5) and (6) are also described as Compounds (2), (3), (4), (5) and (6).

Various kinds of substituents shown in Compounds (1) to (6) are as mentioned below.

As $R^1$, an alkyl group having 1 to 4 carbon atoms, a halogen atom and a hydrogen atom can be mentioned.

As the alkyl group, there may be mentioned those of straight or branched; and preferably $CH_3$ and $C_2H_5$.

As the halogen atom, there may be mentioned a chlorine atom, an iodine atom, a bromine atom and a fluorine atom; and preferably a chlorine atom.

As $R^2$, a halogen atom, an alkyl group having 1 to 4 carbon atoms and a hydrogen atom can be mentioned.

As the halogen atom, there may be mentioned a chlorine atom, an iodine atom, a bromine atom and a fluorine atom; and preferably a chlorine atom.

As the alkyl group, there may be mentioned those of straight or branched; and preferably $CH_3$ and $C_2H_5$.

As $R^3$, an alkyl group having 1 to 8 carbon atoms, a haloalkyl group having 1 to 4 carbon atoms, a substituted or unsubstituted benzoylamino group, a substituted or unsubstituted phenoxy group, an alkenyloxy group having 2 to 5 carbon atoms, an alkynyloxy group having 2 to 5 carbon atoms, a cycloalkyl-carbonylamino group having 4 to 9 carbon atoms, a halo-alkylcarbonyl amino group having 2 to 5 carbon atoms, a cyano group, a formyl group, an acyl group having 2 to 5 carbon atoms, a hydrogen atom or an alkylsulfonyl group having 1 to 4 carbon atoms can be mentioned.

As the alkyl group as a substituent, those of straight or branched may be mentioned, and preferably those having 1 to 6 carbon atoms, more preferably $CH_3$, $C_2H_5$, n-$C_3H_7$, i-$C_3H_7$, n-$C_4H_9$, s-$C_4H_9$ and t-$C_4H_9$. And, the number of the substituent and the position thereof are not particularly limited, and the position is preferably 2, 3 or 4-position when m=1, and 2 and/or 4-position when m=2.

As the haloalkyl group as a substituent, there may be mentioned those in which the alkyl is straight or branched and having a halogen atom such as a chlorine atom, an iodine atom, a bromine atom and a fluorine atom, preferably having a fluorine atom, more preferably $CF_3$. And, the number of the substituent and the position thereof are not particularly limited, and the position is preferably 3 or 4-position when m=1, and 3 and/or 4-position when m=2.

As the halogen atom as a substituent, there may be mentioned a chlorine atom, an iodine atom, a bromine atom and a fluorine atom, etc., and preferably a chlorine atom, a bromine atom and a fluorine atom. And, the number of the substituent and the position thereof are not particularly limited, and the position is preferably 3 or 4-position when m=1, and 2 and/or 6-position when m=2.

The number of the nitro group as a substituent and the position thereof are not particularly limited, and the position is preferably 3 or 4-position when m=1, and 2 and/or 6-position when m=2.

As the alkoxy group as a substituent, there may be mentioned those of straight or branched, and preferably $OCH_3$. And, the number of the substituent and the position thereof are not particularly limited, and the position is preferably 2 or 3-position when m=1, and 2 and/or 6-position when m=2.

As the benzoylamino group as a substituent, there may be mentioned an unsubstituted one or those having a halogen atom as a substituent. And, as the halogen atom, there may be mentioned a chlorine atom, an iodine atom, a bromine atom and a fluorine atom, preferably a fluorine atom. And, the number of the substituent and the position thereof are not particularly limited, and preferably 4-position.

The number of the benzoylamino group as a substituent and the position thereof are not particularly limited, and the position is preferably 3-position when m=1.

As the phenoxy group as a substituent, there may be mentioned an unsubstituted one or those having a nitro group as a substituent. And, the number of the nitro group and the position thereof are not particularly limited, and preferably 4-position.

As the alkenyloxy group as a substituent, there may be mentioned those of straight or branched, and preferably those having 3 carbon atoms, more preferably $OCH_2CH{=}CH_2$. And, the number of the substituent and the position thereof are not particularly limited, and the position is preferably 4-position when m=1.

As the alkynyloxy group as a substituent, there may be mentioned those of straight or branched, and preferably those having 3 carbon atoms, more preferably $OCH_2C{\equiv}CH$. And, the number of the substituent and the position thereof are not particularly limited, and the position is preferably 4-position when m=1.

As the cycloalkylcarbonylamino group as a substituent, there may be mentioned a carbonylamino group having a cycloalkyl group with a carbon number of 3 to 8. And, as the cycloalkyl group, it is preferably a cyclopropyl group. And, the number of the substituent and the position thereof are not particularly limited, and the position is preferably 4-position when m=1.

The number of the cyano group as a substituent and the position thereof are not particularly limited, and the position is preferably 3-position when m=1.

The haloalkylcarbonylamino group as a substituent is a carbonylamino group having a straight or branched haloalkyl group with a carbon number of 1 to 4. As the halogen portion of the haloalkyl group, there may be mentioned a chlorine atom, an iodine atom, a bromine atom and a fluorine atom, etc., and preferably a chlorine atom. As the most preferred haloalkyl portion, there may be mentioned $-CH_2Cl$. And, the number of the substituent and the position thereof are not particularly limited, and the position is preferably 4-position when m=1.

The number of the substituent (s) and the position of the cyano group and the formyl group are not particularly limited, and the position is preferably 4-position when m=1.

The acyl group having 2 to 5 carbon atoms is those having a straight or branched alkyl group with 1 to 4 carbon atoms, and the alkyl portion is preferably $CH_3$. And, the number of the substituent and the position thereof are not particularly limited, and the position is preferably 4-position when m=1.

As the $C_{1-4}$ alkylsulfonyl group, it is an alkyl group having a straight or branched alkyl group with a carbon number of 1 to 4, and the alkyl portion is preferably $CH_3$. And, the number of the substituent and the position thereof are not particularly limited, and the position is preferably 4-position when m=1.

M is preferably 0 to 2.

As Compound (1), those in which the above-mentioned various kinds of substituents are combined may be mentioned, and those which are preferred in view of chemical effects are as mentioned below.

(1) A compound wherein $R^1$ is an alkyl group having 1 to 4 carbon atoms, $R^2$ is a halogen atom, n is 2 and $(R^3)_m$ is an alkyl group having 1 to 8 carbon atoms.
(2) A compound wherein $R^1$ is an alkyl group having 1 to 4 carbon atoms, $R^2$ is a halogen atom, n is 1 and $(R^3)_m$ is an alkyl group having 1 to 8 carbon atoms.
(3) A compound wherein $R^1$ is an alkyl group having 1 to 4 carbon atoms, $R^2$ is a halogen atom, n is 1 and $(R^3)_m$ is a haloalkyl group having 1 to 4 carbon atoms.
(4) A compound wherein $R^1$ is an alkyl group having 1 to 4 carbon atoms, $R^2$ is a halogen atom, n is 2 and $(R^3)_m$ is a haloalkyl group having 1 to 4 carbon atoms.
(5) A compound wherein $R^1$ is an alkyl group having 1 to 4 carbon atoms, $R^2$ is a halogen atom, n is 1 and $(R^3)_m$ is a halogen atom.
(6) A compound wherein $R^1$ is an alkyl group having 1 to 4 carbon atoms, $R^2$ is a halogen atom, n is 2 and $(R^3)_m$ is a halogen atom.
(7) A compound wherein $R^1$ is a halogen atom, $R^2$ is an alkyl group having 1 to 4 carbon atoms, n is 1 and $(R^3)_m$ is an alkyl group having 1 to 8 carbon atoms.
(8) A compound wherein $R^1$ is a halogen atom, $R^2$ is a hydrogen atom, n is 2 and $(R^3)_m$ is an alkyl group having 1 to 8 carbon atoms.
(9) A compound wherein $R^1$ is a halogen atom, $R^2$ is an alkyl group having 1 to 4 carbon atoms, n is 2 and $(R^3)_m$ is an alkyl group having 1 to 8 carbon atoms.
(10) A compound wherein $R^1$ is an alkyl group having 1 to 4 carbon atoms, $R^2$ is a halogen atom, n is 1 and $(R^3)_m$ is (an alkyl group having 1 to 4 carbon atoms)$_2$.
(11) A compound wherein $R^1$ is an alkyl group having 1 to 4 carbon atoms, $R^2$ is a halogen atom, n is 2 and $(R^3)_m$ is (an alkyl group having 1 to 4 carbon atoms)$_2$.
(12) A compound wherein $R^1$ is an alkyl group having 1 to 4 carbon atoms, $R^2$ is a halogen atom, n is 1 and $(R^3)_m$ forms a fused ring with a carbon atom to which it is bonded.
(13) A compound wherein $R^1$ is an alkyl group having 1 to 4 carbon atoms, $R^2$ is a halogen atom, n is 2 and $(R^3)_m$ forms a fused ring with a carbon atom to which it is bonded.
(14) A compound wherein $R^1$ is an alkyl group having 1 to 4 carbon atoms, $R^2$ is a halogen atom, n is 1 and $(R^3)_m$ is a nitro group.
(15) A compound wherein $R^1$ is an alkyl group having 1 to 4 carbon atoms, $R^2$ is a halogen atom, n is 2 and $(R^3)_m$ is a nitro group.
(16) A compound wherein $R^1$ is an alkyl group having 1 to 4 carbon atoms, $R^2$ is a halogen atom, n is 2 and $(R^3)_m$ is an alkoxy group having 1 to 4 carbon atoms.
(17) A compound wherein $R^1$ is an alkyl group having 1 to 4 carbon atoms, $R^2$ is a halogen atom, n is 1 and $(R^3)_m$ is an alkoxy group having 1 to 4 carbon atoms.
(18) A compound wherein $R^1$ is an alkyl group having 1 to 4 carbon atoms, $R^2$ is a halogen atom, n is 2 and $(R^3)_m$ is a benzoylamino group.
(19) A compound wherein $R^1$ is an alkyl group having 1 to 4 carbon atoms, $R^3$ is a halogen atom, n is 1 and $(R^3)_m$ is a benzoylamino group.
(20) A compound wherein $R^1$ is an alkyl group having 1 to 4 carbon atoms, $R^2$ is a halogen atom, n is 2 and $(R^3)_m$

is (an alkoxy group having 1 to 4 carbon atoms)$_2$.

(21) A compound wherein $R^1$ is an alkyl group having 1 to 4 carbon atoms, $R^2$ is a halogen atom, n is 1 and $(R^3)_m$ is (an alkoxy group having 1 to 4 carbon atoms)$_2$.

(22) A compound wherein $R^1$ is an alkyl group having 1 to 4 carbon atoms, $R^2$ is a halogen atom, n is 1 and $(R^3)_m$ is (a halogenatom)$_2$.

(23) A compound wherein $R^1$ is an alkyl group having 1 to 4 carbon atoms, $R^2$ is a halogen atom, n is 2 and $(R^3)_m$ is an alkenyloxy group having 2 to 5 carbon atoms.

(24) A compound wherein $R^1$ is an alkyl group having 1 to 4 carbon atoms, $R^2$ is a halogen atom, n is 2 and $(R^3)_m$ is an alkynyloxy group having 2 to 5 carbon atoms.

(25) A compound wherein $R^1$ is a hydrogen atom, $R^2$ is a halogen atom, n is 2 and $(R^3)_m$ is a haloalkyl group having 1 to 4 carbon atoms.

(26) A compound wherein $R^1$ is a halogen atom, $R^2$ is a hydrogen atom, n is 1 and $(R^3)_m$ is a haloalkyl group having 1 to 4 carbon atoms.

(27) A compound wherein $R^1$ is a halogen atom, $R^2$ is an alkyl group having 1 to 4 carbon atoms, n is 2 and $(R^3)_m$ is a halogen atom.

(28) A compound wherein $R^1$ is an alkyl group having 1 to 4 carbon atoms, $R^2$ is a halogen atom, n is 1 and $(R^3)_m$ is (a nitro group, a haloalkyl group having 1 to 4 carbon atoms).

(29) A compound wherein $R^1$ is an alkyl group having 1 to 4 carbon atoms, $R^2$ is a halogen atom, n is 2 and $(R^3)_m$ is a phenoxy group.

(30) A compound wherein $R^1$ is a halogen atom, $R^2$ is a hydrogen atom, n is 1 and $(R^3)_m$ is an alkyl group having 1 to 4 carbon atoms.

(31) A compound wherein $R^1$ is an alkyl group having 1 to 4 carbon atoms, $R^2$ is a halogen atom, n is 2 and $(R^3)_m$ is a cyano group.

(32) A compound wherein $R^1$ is an alkyl group having 1 to 4 carbon atoms, $R^2$ is a halogen atom, n is 1 and $(R^3)_m$ is a cyano group.

(33) A compound wherein $R^1$ is an alkyl group having 1 to 4 carbon atoms, $R^2$ is a halogen atom, n is 1 and $(R^3)_m$ is a formyl group.

(34) A compound wherein $R^1$ is an alkyl group having 1 to 4 carbon atoms, $R^2$ is a halogen atom, n is 2 and $(R^3)_m$ is a formyl group.

(35) A compound wherein $R^1$ is a halogen atom, $R^2$ is a hydrogen atom, n is 2 and $(R^3)_m$ is a halogen atom.

(36) A compound wherein $R^1$ is a halogen atom, $R^2$ is a hydrogen atom, n is 1 and $(R^3)_m$ is a halogen atom.

(37) A compound wherein $R^1$ is a halogen atom, $R^2$ is a hydrogen atom, n is 2 and $(R^3)_m$ is a haloalkyl group having 1 to 4 carbon atoms.

(38) A compound wherein $R^1$ is a halogen atom, $R^2$ is a hydrogen atom, n is 2 and $(R^3)_m$ is an acyl group having 2 to 5 carbon atoms.

(39) A compound wherein $R^1$ is a halogen atom, $R^2$ is a hydrogen atom, n is 2 and $(R^3)_m$ forms a fused ring with a carbon atom to which it is bonded.

(40) A compound wherein $R^1$ is an alkyl group having 1 to 4 carbon atoms, $R^2$ is a halogen atom, n is 2 and $(R^3)_m$ is an acyl group having 2 to 5 carbon atoms.

(41) A compound wherein $R^1$ is an alkyl group having 1 to 4 carbon atoms, $R^2$ is a halogen atom, n is 1 and $(R^3)_m$ is an acyl group having 2 to 5 carbon atoms.

As $R^1$ to $R^3$, m and n of Compound (1) shown by (1) to (41) comprising a preferred combination thereof, there may be exemplified by those shown as a preferred one, and those shown as a more preferred one described in the above explanation.

As Compound (4), preferably those having $R^1$ to $R^3$, m and n corresponding to those of Compound (1) as mentioned above may be mentioned.

Compound (1) can be synthesized according to the preparation method 1 or 2.

(Preparation method 1)

Compound (1) can be synthesized, as shown below, by reacting Compound (2) and Compound (3) in a solvent, and the resulting Compound (4) is oxidized. Also, the reaction of Compound (2) and Compound (3) can be promoted by using a base.

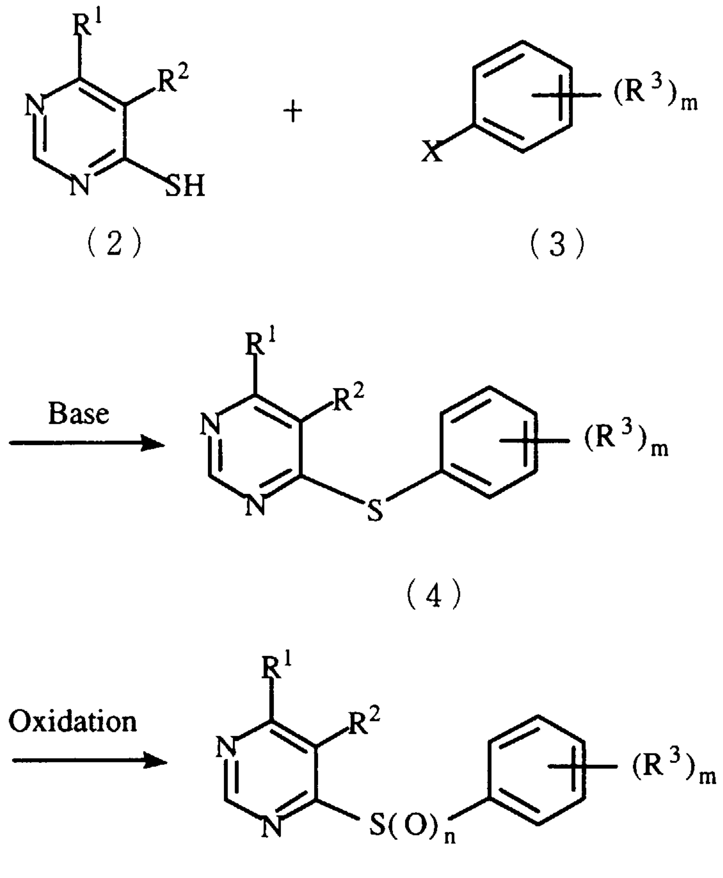


(2) (3)

(4)

(1)

wherein $R^1$ to $R^3$, m, n and X have the same meanings as defined above.

As the kind of the solvent, it is not particularly limited so long as it does not participate in the present reaction directly, and there may be mentioned, for example, ketones, amides, nitriles, organic bases, 1,3-dimethyl-2-imidazolidinone, dimethylsulfoxide, and a mixture of the above solvents.

As the ketones, there may be mentioned acetone, methyl ethyl ketone, etc.

As the amides, there may be mentioned N,N-dimethylformamide, N,N-dimethylacetamide, etc.

As the nitriles, there may be mentioned acetonitrile, propionitrile, etc.

As the organic bases, there may be mentioned triethylamine, pyridine, N,N-dimethylaniline, 1,8-diazabicyclo[5.4.0]-undeca-7-ene, etc.

An amount of the solvent to be used can be used so as to become the total amount used of Compounds (2) and (3) being 5 to 80 % by weight, preferably 10 to 70 % by weight.

The kind of the base is not particularly specified, and there may be mentioned, for example, an organic base such as triethylamine, pyridine, 4-N,N-dimethylaminopyridine, N,N-dimethylaniline, 1,8-diazabicyclo[5.4.0]undeca-7-ene, etc.; alkali metal alkoxides; an inorganic base such as potassium t-butoxide, potassium carbonate, sodium carbonate, potassium hydrogen carbonate, sodium hydrogen carbonate, etc.; lithium diisopropylamide, bistrimethylsilyl lithium amide, etc.

An amount of the base to be used is 0.001 to 5-fold moles based on Compound (2).

A reaction temperature is not particularly limited, and it is within the temperature range of room temperature to 200°C, preferably 50 to 150°C.

A reaction time of Compound (2) and Compound (3) may vary depending on the concentration and the temperature as mentioned above, and is generally 0.1 to 100 hours, preferably 0.3 to 24 hours.

An amount of the starting compound to be used is 0.5 to 50-fold moles of Compound (3) based on the amount

of Compound (2), preferably 0.8 to 10-fold moles.

As an oxidizing agent, there may be mentioned peroxides such as hydrogen peroxide, peracetic acid, perbenzoic acid, m-chloroperbenzoic acid, etc.; sodium metaperiodate; hydroperoxide; ozone; selenium dioxide; chromic acid; dinitrogen tetroxide; acyl nitrate; iodine; N-bromosuccinimide; iodosyl benzene; sulfuryl chloride; hydrated silica gel; t-butyl hypochlorite; oxone (potassium peroxymonosulfate available from Aldrich Co.), etc.

An amount of the oxidizing agent to be used is 0.01 to 5-fold moles based on the amount of Compound (4).

A reaction temperature is not particularly limited, and it is within the temperature range of -80 to 100°C, preferably -60 to 100°C.

An oxidation reaction time of Compound (4) may vary depending on the concentration and the temperature as mentioned above, and is generally 0.1 to 100 hours, preferably 0.1 to 24 hours.

Compound (2) may be used, for example, by preparing it according to the method described in J. Org. Chem., 1164 (1962), J. Am. Chem. Soc., 2256 (1855), etc., as far as it is not commercially available.

As Compound (2), there may be mentioned Compound (2) comprising the substituents $R^1$ and $R^2$ corresponding to Compounds 1 to 83 described in Table 1 mentioned hereinbelow (which are referred to as Compounds $(2)_1$ to $(2)_{83}$. For example, Compound $(2)_1$ is $R^1$ in the formula shown by Compound (2) is $C_2H_5$ and $R^2$ is Cl.).

Compound (3) may be used, for example, by preparing it according to the method described in G.C. Finger, J.L. Finnerty, Biochem. Prep. 3, 120 (1953), etc., as far as it is not commercially available.

As Compound (3), there may be mentioned Compound (3) comprising the substituent $(R^3)_m$ corresponding to Compounds 1 to 83 described in Table 1 mentioned hereinbelow (which are referred to as Compounds $(3)_1$ to $(3)_{83}$. For example, Compound $(3)_1$ is $(R^3)_m$ in the formula shown by Compound (3) is 4-t-$C_4H_9$ and X is a halogen atom.).

(Preparation method 2)

Compound (1) can be synthesized, as shown below, by reacting Compound (5) and Compound (6) in a solvent, and the resulting Compound (4) is oxidized. Also, the reaction of Compound (5) and Compound (6) can be promoted by using a base.

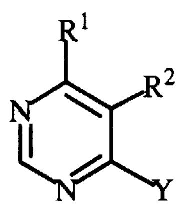

(5)

+

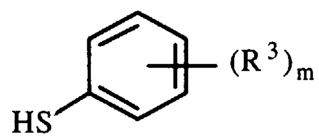

(6)

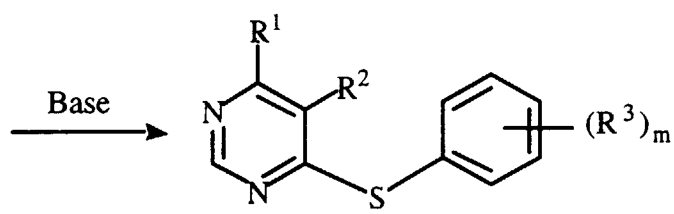


(4)

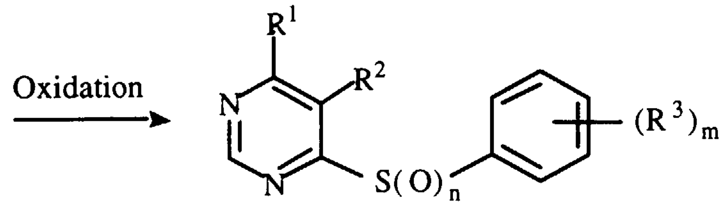


(1)

wherein $R^1$ to $R^3$, m, n and Y have the same meanings as defined above.

This reaction can be carried out by using Compound (5) in place of Compound (2) in Preparation method 1 and Compound (6) in place of Compound (3), and employing the same method as in Preparation method 1 to effect synthesis.

Compound (5) may be used, for example, by preparing it according to the method described in J. Cem. Soc., 3478 (1955), etc., as far as it is not commercially available.

As Compound (5), there may be mentioned Compound (5) comprising the substituents $R^1$ and $R^2$ corresponding to Compounds 1 to 83 described in Table 1 mentioned hereinbelow (which are referred to as Compounds $(5)_1$ to $(5)_{83}$. For example, Compound $(5)_1$ is $R^1$ in the formula shown by Compound (5) is $C_2H_5$ and $R^2$ is Cl.).

Compound (6) may be used, for example, by preparing it according to the method described in J. Org. Chem., 3980 (1966), J. Chem. Soc., 1465 (1946), etc., as far as it is not commercially available.

As Compound (6), there may be mentioned Compound (6) comprising the substituent $(R^3)_m$ corresponding to Compounds 1 to 83 described in Table 1 mentioned hereinbelow (which are referred to as Compounds $(6)_1$ to $(6)_{83}$. For example, Compound $(6)_1$ is that $(R^3)_m$ in the formula shown by Compound (6) is 4-t-$C_4H_9$ and X is a halogen atom.).

The desired compound (1) prepared as mentioned above can be subjected to, after completion of the reaction, a usual post-treatment such as extraction, condensation, filtration, etc., and, if necessary, optionally purified by effecting known means such as recrystallization, various kinds of chromatography, etc.

As Compound (1) obtained as mentioned above, there may be mentioned, for example, Compounds 1 to 83 described in Table 1 mentioned hereinbelow.

Also, as Compound (4), there may be mentioned, for example, Compounds (4-1) to (4-12) described in Table 3 mentioned hereinbelow.

As agricultural and horticultural diseases on which a controlling effect by Compound (1) of the present invention can be observed, there may be mentioned, for example, cucumber downy mildew, cucumber gray mold, rice blast, wheat brown rust, barley powdery mildew, tomato plague, rice "Bakanae" disease, rice sheath blight, etc.

The agricultural and horticultural fungicide of the present invention contains one kind or more of specific compound belonging to the category of Compound (1) as an effective ingredient.

Compound (1) may be used singly, but usually, it is preferred to formulate a carrier, surfactant, dispersant, auxiliary, etc. (for example, it is prepared as a composition such as dust powder, an emulsifiable concentrate, a fine granule, a granule, a wettable powder, an oily suspension, an aerosol, etc.) according to the conventionally known method.

As the carrier, there may be mentioned, for example, a solid carrier such as bentonite, clay, kaolin, diatomaceous earth, white carbon, vermiculite, calcium hydroxide, siliceous sand, ammonium sulfate, urea, etc., a liquid carrier such as hydrocarbon (kerosine, mineral oil, etc.), aromatic hydrocarbon, (benzene, toluene, xylene, etc.), chlorinated hydrocarbon (chloroform, carbon tetrachloride, etc.), ethers (dioxane, tetrahydrofuran, etc.), ketones (acetone, cyclohexanone, isophorone, etc.), esters (ethyl acetate, ethyleneglycol acetate, dibutyl maleate, etc.), alcohols (methanol, n-hexanone, ethylene glycol, etc.), polar solvent (dimethylformamide, dimethylsulfoxide, etc.), water, etc.; a gas carrier such as air, nitrogen, a carbonic acid gas, fleone, etc. (in this case, mixture spreading can be carried out), and the like.

As the surfactant and dispersant which can be used for improving attachment of the present chemical to and absorption thereof in animals and plants, and improving characteristics such as dispersion, emulsification and spreading of the chemical, there may be mentioned, for example, alcohol sulfates, alkylsulfonate, lignosulfonate and polyoxyethylene glycol ether. Further, for improving properties of its formulation, for example, carboxymethyl cellulose, polyethylene glycol and gum arabic can be used as an auxiliary.

In preparation of the present chemical, the above carrier, surfactant, dispersant and auxiliary can be used singly or in a suitable combination, respectively, depending on the respective purposes.

When the compound (I) of the present invention is made into formulations, the concentration of the active ingredient is generally 1 to 50 % by weight in an emulsifiable concentrate, generally 0.3 to 25 % by weight in a dustable powder, generally 1 to 90 % by weight in a wettable powder, generally 0.5 to 5 % by weight in a granule, generally 0.5 to 5 % by weight in an oily suspension, and generally 0.1 to 5 % by weight in an aerosol.

These formulations can be provided for various uses by diluting them to have a suitable concentration and spraying them to stems and leaves of plants, soil and paddy field surface, or by applying them directly thereto, depending on the purposes.

Examples

In the following, the present invention is explained in detail by referring to Reference examples and Examples.

These Examples do not limit the scope of the present invention.

Reference example 1 (Synthesis of Compound (2))

(1) Synthesis of 5-chloro-6-methyl-4-mercaptopyrimidine (Compound $(2)_4$)

In water (100 ml) were dissolved 4,5-dichloro-6-methyl-pyrimidine (12.8 g) and 70% sodium hydrosulfide (6.3 g), and the mixture was stirred at room temperature for 4 hours.

After completion of the reaction, the reaction mixture was filtered, and the residue was washed with water and then with hexane and dried to obtain 10.0 g of the desired compound (m.p. 97 to 98 °C).

(2) Synthesis of other compounds (2) in Table 1

In accordance with the method described in the above-mentioned (1), other compounds (2) in Table 1 were synthesized.

Example 1 (Synthesis of Compound (1))

(1) Synthesis of 5-chloro-6-methyl-(4-nitrophenylsulfinyl)pyrimidine (Compound 25)

In dimethylformamide (40 ml) was dissolved 5-chloro-6-methyl-4-mercaptopyrimidine (3.2 g), then, 4-fluoronitrobenzen (2.8 g) and potassium carbonate (3.1 g) were added to the above mixture, and the resulting mixture was stirred under nitrogen stream and heating for about 2 hours.

After completion of the reaction, the reaction mixture was cooled to room temperature and subjected to extraction by adding toluene (100 ml) and water (120 ml). These liquids were separated and the organic layer was dried over magnesium sulfate, and then, concentrated under reduced pressure.

The resulting residue was purified by silica gel column chromatography (Wako gel C-200, eluted by toluene) to obtain 5.2 g (Yield: 92 %) of 5-chloro-6-methyl-4-(4-nitrophenylthio)pyrimidine.

In methylene chloride (100 ml) was dissolved 3-chloroperbenzoic acid (3.5 g), and to the solution was added dropwise a methylene chloride solution (20 ml) of 5-chloro-6-methyl-4-(4-nitrophenylthio)pyrimidine, and the mixture was returned to room temperature and stirred for 2 hours.

After completion of the reaction, the reaction mixture was subjected to extraction by adding water (100ml) and 2N-sodium hydroxide solution (20 ml). These liquids were separated, and the organic layer was dried over magnesium sulfate, and then, concentrated under reduced pressure.

The resulting residue was purified by silica gel column chromatography (Wako gel C-200, eluted by toluene:ethyl acetate = 4:1) to obtain 1.9 g (Yield: 64 %) of the desired compound.

- m.p. 172 to 173°C
- $^1$H-NMR ($CDCl_3$, $\delta$ ppm)
  2.67 (s, 3H), 8.10 (d, 2H), 8.36 (d, 2H), 9.10 (s, 1H)

(2) Synthesis of 6-chloro-4-(3-trifluoromethylphenylsulfenyl)pyrimidine (Compound (40)).

In dimethylformamide (20 ml) were dissolved 4,6-dichloropyrimidine (0.74 g) and 3-trifluoromethylthiophenol (1.0 g), and potassium carbonate (0.8 g) was added to the solution and the solution was stirred under nitrogen stream and heating for about 2 hours.
After completion of the reaction, the reaction mixture was cooled to room temperature and subjected to extraction by adding toluene (50 ml) and water (70 ml). These liquids were separated and the organic layer was dried over magnesium sulfate, and then, concentrated under reduced pressure.

The resulting residue was purified by silica gel column chromatography (Wako gel C-200, eluted by toluene) to obtain 0.8 g (Yield: 55 %) of 6-chloro-4-(3-trifluoromethylphenylthio)pyrimidine.

A methylene chloride solution (10 ml) of 6-chloro-4-(3-trifluoromethylphenylthio)pyrimidine obtained as mentioned above was added dropwise to methylene chloride (10 ml) of 3-chloroperbenzoic acid (1.9 g) under ice-cooling, and then the reaction mixture was returned to room temperature and stirred for 2 hours.

After completion of the reaction, the reaction mixture was subjected to extraction by adding water (80 ml) and 2N-sodium hydroxide solution (10ml). These liquids were separated, and the organic layer was dried over magnesium sulfate, and then, concentrated under reduced pressure.

The resulting residue was purified by silica gel column chromatography (Wako gel C-200, eluted by toluene:ethyl acetate = 4:1) to obtain 0.6 g (Yield: 70 %) of the desired compound.

- m.p. 97 to 98°C
- $^1$H-NMR ($CDCl_3$, $\delta$ ppm)
  7.75 (t, 1H), 8.00 (d, 1H), 8.20 (s, 1H), 8.30 (s, 1H), 9.05 (s, 1H)

(3) Synthesis of 6-chloro-4-(4-chlorophenylsulfinyl)pyrimidine (Compound 53)

In methylene chloride (80ml) was dissolved 3-chloroperbenzoic acid (1.3 g), and after ice-cooling, methylene chloride (20 ml) containing 6-chloro-4-(4-chlorophenylthio)pyrimidine (1.0 g) was added dropwise thereto, and the reaction mixture was returned to room temperature and stirred for 2 hours.

After completion of the reaction, the reaction mixture was subjected to extraction by adding water (100 ml) and 2 N-sodium hydroxide aqueous solution (20 ml). These liquids were separated, and the organic layer was dried over magnesium sulfate, and then, concentrated under reduced pressure.

The resulting residue was purified by silica gel column chromatography (Wako gel C-200, eluted by toluene:ethyl acetate = 4:1) to obtain 0.8 g (Yield: 80 %) of the desired compound.

- $^1$H-NMR ($CDCl_3$, $\delta$ ppm)
  7.40 (d, 2H), 7.80 (d, 2H), 8.05 (d, 1H), 8.95 (s, 1H)

(4) Synthesis of 6-chloro-4-(4-chlorophenylsulfonyl)pyrimidine (Compound 53)

In methylene chloride (100 ml) was dissolved 3-chloroperbenzoic acid (2.7 g), and under ice-cooling, methylene chloride (20 ml) containing 6-chloro-4-(4-chlorophenylthio)pyrimidine (1.0 g) was added dropwise thereto. Then, the mixture was returned to room temperature and stirred for 2 hours.

After completion of the reaction, the reaction mixture was subjected to extraction by adding water (100 ml) and 2N-sodium hydroxide aqueous solution (30 ml). These liquids were separated, and the organic layer was dried over magnesium sulfate, and then, concentrated under reduced pressure.

The resulting residue was purified by silica gel column chromatography (Wako gel C-200, eluted by toluene:ethyl acetate = 4:1) to obtain 1.0 g (Yield: 85 %) of the desired compound.

- $^1$H-NMR ($CDCl_3$, $\delta$ ppm)
  7.55 (d, 2H), 8.05 (d, 2H), 8.15 (s, 1H), 9.05 (s, 1H)

(5) Synthesis of other Compounds (1) in Table 1

In accordance with the methods described in (1) to (4) mentioned above, other Compounds (1) in Table 1 were synthesized.

Compounds (1) synthesized as mentioned above and their physical properties are shown in Tables 1 and 2.

Table 1

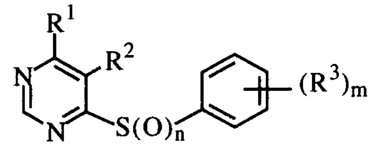
(1)

| Compound | $R^1$ | $R^2$ | n | $(R^3)_m$ | Physical property |
|---|---|---|---|---|---|
| 1 | $C_2H_5$ | Cl | 2 | 4-t-$C_4H_9$ | Paste state |
| 2 | $C_2H_5$ | Cl | 1 | 4-t-$C_4H_9$ | m.p. 101~104℃ |
| 3 | $C_2H_5$ | Cl | 1 | 4-$CF_3$ | m.p. 110~112℃ |
| 4 | $CH_3$ | Cl | 1 | 4-$CH_3$ | m.p. 127~128℃ |
| 5 | $CH_3$ | Cl | 1 | 3-$CF_3$ | $n_D^{20}$ 1.5682 |
| 6 | $CH_3$ | Cl | 2 | 3-$CF_3$ | m.p. 132~134℃ |
| 7 | $CH_3$ | Cl | 1 | 3-Cl | m.p. 99~101℃ |
| 8 | $CH_3$ | Cl | 2 | 3-Cl | m.p. 106~108℃ |
| 9 | $CH_3$ | Cl | 2 | 3-Br | m.p. 124~125℃ |
| 10 | $CH_3$ | Cl | 1 | 3-Br | m.p. 130~132℃ |
| 11 | $CH_3$ | Cl | 2 | 3-$CH_3$ | m.p. 89~ 91℃ |
| 12 | $CH_3$ | Cl | 1 | 3-$CH_3$ | m.p. 80~ 81℃ |
| 13 | Cl | $C_2H_5$ | 1 | 4-t-$C_4H_9$ | $n_D^{20}$ 1.5842 |

m.p.: melting point

Table 1 (contd)

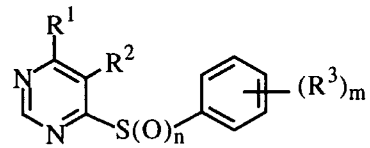 (1)

| Compound | $R^1$ | $R^2$ | n | $(R^3)_m$ | Physical property |
|---|---|---|---|---|---|
| 14 | Cl | H | 2 | 4-t-$C_4H_9$ | m.p. 68~ 69℃ |
| 15 | Cl | $C_2H_5$ | 2 | 4-t-$C_4H_9$ | m.p. 83~ 85℃ |
| 16 | $CH_3$ | Cl | 1 | 2-$CH_3$, 4-$CH_3$ | $n_D^{20}$ 1.6158 |
| 17 | $CH_3$ | Cl | 2 | 2-$CH_3$, 4-$CH_3$ | m.p. 100~103℃ |
| 18 | $CH_3$ | Cl | 1 | 3- 4- 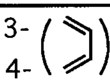 | see Table 2 |
| 19 | $CH_3$ | Cl | 2 | 3- 4- 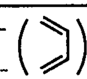 | m.p. 176~178℃ |
| 20 | $CH_3$ | Cl | 1 | 4-Cl | m.p. 129~130℃ |
| 21 | $CH_3$ | Cl | 2 | 4-Cl | m.p. 139~140℃ |
| 22 | $CH_3$ | Cl | 2 | 4-$CH_3$ | m.p. 111~112℃ |
| 23 | $CH_3$ | Cl | 1 | 2-$CH_3$ | see Table 2 |
| 24 | $CH_3$ | Cl | 2 | 2-$CH_3$ | m.p. 142~145℃ |
| 25 | $CH_3$ | Cl | 1 | 4-$NO_2$ | m.p. 172~173℃ |
| 26 | $CH_3$ | Cl | 2 | 4-$NO_2$ | m.p. 186~187℃ |

Table 1 (contd)

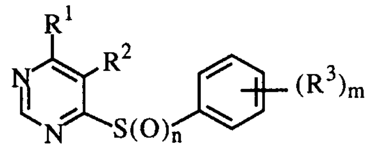 (1)

| Compound | $R^1$ | $R^2$ | n | $(R^3)_m$ | Physical property |
|---|---|---|---|---|---|
| 27 | $CH_3$ | Cl | 2 | 2-$OCH_3$ | m.p. 133~135℃ |
| 28 | $CH_3$ | Cl | 1 | 2-$OCH_3$ | m.p. 107~109℃ |
| 29 | $CH_3$ | Cl | 2 | 3-$OCH_3$ | see Table 2 |
| 30 | $CH_3$ | Cl | 1 | 3-$OCH_3$ | m.p. 74~ 77℃ |
| 31 | $CH_3$ | Cl | 2 | 3-NHCO 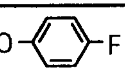 F | m.p. 165~167℃ |
| 32 | $CH_3$ | Cl | 1 | 3-NHCO 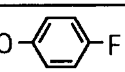 F | m.p. 180~182℃ |
| 33 | $C_2H_5$ | Cl | 2 | 2-$OCH_3$, 6-$OCH_3$ | $n_D^{14}$ 1.5844 |
| 34 | $C_2H_5$ | Cl | 1 | 2-$OCH_3$, 6-$OCH_3$ | $n_D^{14}$ 1.6010 |
| 35 | $C_2H_5$ | Cl | 2 | 2-Cl, 6-Cl | m.p. 127~129℃ |
| 36 | $CH_3$ | Cl | 2 | 4-O 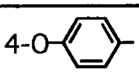 $NO_2$ | m.p. 121~122℃ |
| 37 | $CH_3$ | Cl | 2 | 4-$OCH_2CH=CH_2$ | m.p. 87~ 88℃ |
| 38 | $CH_3$ | Cl | 2 | 4-$OCH_2C≡CH$ | m.p. 125~126℃ |
| 39 | $CH_3$ | Cl | 1 | 3-$NO_2$ | see Table 2 |

Table 1 (contd)

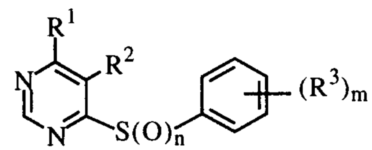

(1)

| Compound | $R^1$ | $R^2$ | n | $(R^3)_m$ | Physical property |
|---|---|---|---|---|---|
| 40 | H | Cl | 2 | 3-$CF_3$ | m.p. 97~ 98℃ |
| 41 | Cl | H | 1 | 3-$CF_3$ | m.p. 62~ 65℃ |
| 42 | Cl | $C_2H_5$ | 2 | 3-Cl | m.p. 85~ 86℃ |
| 43 | Cl | $C_2H_5$ | 1 | 3-Cl | $n_D^{20}$ 1.6199 |
| 44 | $CH_3$ | Cl | 1 | 2-$NO_2$, 4-$CF_3$ | see Table 2 |
| 45 | $CH_3$ | Cl | 1 | 3-$CF_3$, 4-$NO_2$ | m.p. 102~103℃ |
| 46 | $CH_3$ | Cl | 1 | 3-CN, 4-$NO_2$ | m.p. 163~164℃ |
| 47 | $CH_3$ | Cl | 2 | 2-$NO_2$, 4-$CF_3$ | see Table 2 |
| 48 | $CH_3$ | Cl | 2 | 4-NHCO 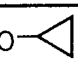 | m.p. 211~212℃ |
| 49 | $CH_3$ | Cl | 2 | 3-$CF_3$, 4-$NO_2$ | m.p. 217~218℃ |
| 50 | $CH_3$ | Cl | 2 | 3-CN, 4-$NO_2$ | m.p. 198~199℃ |
| 51 | $CH_3$ | Cl | 2 | 3-Cl, 4-Cl | m.p. 132~134℃ |
| 52 | $CH_3$ | Cl | 1 | 3-Cl, 4-Cl | m.p. 116~117℃ |

Table 1 (contd)

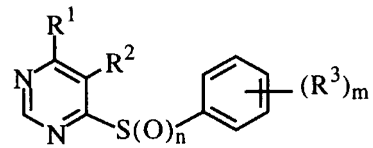

(1)

| Compound | $R^1$ | $R^2$ | n | $(R^3)_m$ | Physical property |
|---|---|---|---|---|---|
| 5 3 | Cl | H | 1 | 4-Cl | see Table 2 |
| 5 4 | Cl | H | 2 | 4-Cl | see Table 2 |
| 5 5 | Cl | H | 2 | 4-$NO_2$ | see Table 2 |
| 5 6 | Cl | H | 1 | 4-$NO_2$ | see Table 2 |
| 5 7 | $CH_3$ | Cl | 2 | 4- 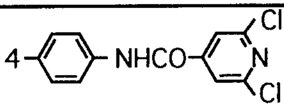 | m.p. 231~233℃ |
| 5 8 | $CH_3$ | Cl | 1 | 4- 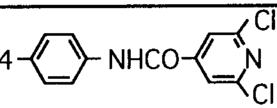 | m.p. 229~232℃ |
| 5 9 | Cl | H | 2 | 4-$CH_3$ | m.p. 119~120℃ |
| 6 0 | Cl | H | 1 | 4-$CH_3$ | $n_D^{20}$ 1.6187 |
| 6 1 | $CH_3$ | Cl | 2 | 4-$NHCOCH_2Cl$ | m.p. 148~149℃ |
| 6 2 | $CH_3$ | Cl | 1 | 4-$NHCOCH_2Cl$ | m.p. 186~188℃ |
| 6 3 | $CH_3$ | Cl | 2 | 4-CN | m.p. 157~161℃ |
| 6 4 | $CH_3$ | Cl | 1 | 4-CN | m.p. 152~156℃ |
| 6 5 | $CH_3$ | Cl | 1 | 4-CHO | m.p. 123~125℃ |

Table 1 (contd)

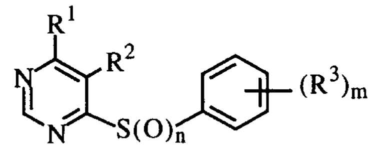 (1)

| Compound | $R^1$ | $R^2$ | n | $(R^3)_m$ | Physical property |
|---|---|---|---|---|---|
| 66 | $CH_3$ | Cl | 2 | 4-CHO | m.p. 140~142℃ |
| 67 | Cl | H | 2 | 4-Br | m.p. 149~150℃ |
| 68 | Cl | H | 1 | 4-Br | m.p. 98~101℃ |
| 69 | Cl | H | 2 | 4-$CF_3$ | m.p. 118~119℃ |
| 70 | Cl | H | 1 | 4-$CF_3$ | m.p. 112~113℃ |
| 71 | Cl | H | 2 | 4-$COCH_3$ | m.p. 110~113℃ |
| 72 | Cl | H | 2 | 3- 4- 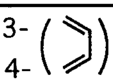 | m.p. 130~134℃ |
| 73 | Cl | H | 2 | 3-Cl | m.p. 118~121℃ |
| 74 | Cl | H | 1 | 3-Cl | $n_D^{25}$ 1.6334 |
| 75 | $CH_3$ | Cl | 2 | 4-$COCH_3$ | m.p. 160~162℃ |
| 76 | $CH_3$ | Cl | 1 | 4-$COCH_3$ | m.p. 70~ 72℃ |
| 77 | Cl | H | 2 | 3-Br | m.p. 128~130℃ |
| 78 | Cl | H | 1 | 3-Br | $n_D^{28}$ 1.6464 |

Table 1 (contd)

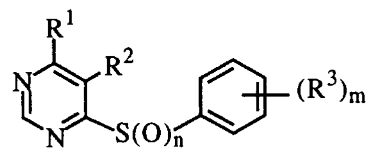

(1)

| Compound | $R^1$ | $R^2$ | n | $(R^3)_m$ | Physical property |
|---|---|---|---|---|---|
| 79 | Cl | H | 1 | 4-$COCH_3$ | |
| 80 | Cl | H | 2 | H | m.p. 108~110℃ |
| 81 | Cl | H | 2 | 4-F | m.p. 154~156℃ |
| 82 | $CH_3$ | H | 1 | 4-$SO_2CH_3$ | m.p. 154~156℃ |
| 83 | $CH_3$ | H | 2 | 4-$SO_2CH_3$ | |

Table 2

| Compound | Physical property : $^1H-NMR$ ($CDCl_3$), (δ ppm) |
|---|---|
| 18 | 2.60 (s, 3H), 7.55~7.65 (m, 2H), 7.80~8.00 (m, 4H), 8.43 (s, 1H), 9.12 (s, 1H) |
| 23 | 2.70 (s, 3H), 7.22~7.45 (m, 3H), 7.80 (d, 1H), 9.10 (s, 1H) |
| 29 | 2.70 (s, 3H), 3.90 (s, 3H), 7.15 (d, 1H), 7.20~7.30 (m, 1H), 7.50 (d, 1H), 7.65 (d, 1H), 8.95 (s, 1H) |
| 39 | 2.65 (s, 3H), 7.85 (t, 1H), 8.10~8.20 (m, 1H), 8.25 (s, 2H), 9.15 (s, 1H) |
| 44 | 2.80 (s, 3H), 8.35 (d, 1H), 8.58~8.75 (m, 3H) |
| 47 | 2.80 (s, 3H), 8.15 (d, 1H), 8.40 (s, 1H), 8.65 (d, 1H), 8.80 (s, 1H) |
| 53 | 7.40 (d, 2H), 7.80 (d, 2H), 8.05 (s, 1H), 8.95 (s, 1H) |
| 54 | 7.55 (d, 2H), 8.05 (d, 2H), 8.15 (s, 1H), 9.05 (s, 1H) |
| 55 | 8.20 (d, 2H), 8.25 (d, 2H), 8.45 (s, 1H), 9.05 (s, 1H) |
| 56 | 8.00 (d, 2H), 8.05 (d, 2H), 8.40 (s, 1H), 8.90 (s, 1H) |

Example 2 (Synthesis of Compound (4))

(1) Synthesis of 6-chloro-4-(4-chlorophenylthio)pyrimidine (Compound (4-1))

In dimethylformamide (15 ml) was dissolved 4,6-dichloropyrimidine (1.5 g), and then, 4-chlorothiophenol (1.6 g) and potassium carbonate (1.6g) were added thereto, and the mixture was stirred under nitrogen stream and heating for about 2 hours.
After completion of the reaction, the reaction mixture was cooled to room temperature and subjected to extraction by adding toluene (70 ml) and water (100 ml). These liquids were separated, and the organic layer was dried over magnesium sulfate, and then, concentrated under reduced pressure.
The resulting residue was purified by silica gel column chromatography (Wako gel C-200, eluted by toluene) to obtain 2.3 g (Yield: 90 %) of the desired compound.

- $^1$H-NMR ($CDCl_3$, $\delta$ ppm)
  6.85 (s, 1H), 7.50 (m, 4H), 8.70 (s, 1H)

(2) Synthesis of other Compounds (4) in Table 3

In accordance with the method described in (1) as mentioned above, other Compounds (4) in Table 3 were synthesized.
Compounds (4) synthesized as mentioned above and their physical properties are shown in Tables 3 and 4.

Table 3

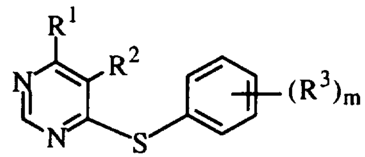

(4)

| Compound | $R^1$ | $R^2$ | $(R^3)_m$ | Physical property |
|---|---|---|---|---|
| 4 − 1 | Cl | H | 4-Cl | see Table 4 |
| 4 − 2 | $CH_3$ | Cl | 3-Cl, 4-Cl | see Table 4 |
| 4 − 3 | Cl | $C_2H_5$ | 3-Cl | see Table 4 |
| 4 − 4 | $CH_3$ | Cl | 2-$NO_2$, 4-$CF_3$ | see Table 4 |
| 4 − 5 | Cl | H | 3-$CF_3$ | see Table 4 |
| 4 − 6 | Cl | H | 2-$OCH_3$ | see Table 4 |
| 4 − 7 | $CH_3$ | Cl | 3-$OCH_3$ | see Table 4 |
| 4 − 8 | Cl | H | 4-t-$C_4H_9$ | see Table 4 |
| 4 − 9 | $CH_3$ | Cl | 3-Br | see Table 4 |
| 4 − 10 | $CH_3$ | Cl | 3-Cl | see Table 4 |
| 4 − 11 | $CH_3$ | Cl | 3-$CF_3$ | see Table 4 |
| 4 − 12 | $CH_3$ | Cl | 4-Cl | see Table 4 |

Table 4

| Compound | Physical property : $^1H-NMR$ ($CDCl_3$), (δ ppm) |
|---|---|
| 4 − 1 | 6.85 (s, 1H), 7.50 (m, 4H), 8.70 (s, 1H) |
| 4 − 2 | 2.50 (s, 3H), 7.35 (m, 1H), 7.55 (d, 1H), 7.65 (d, 1H), 8.60 (s, 1H) |
| 4 − 3 | 1.30 (t, 3H), 2.85 (q, 2H), 7.35~7.45 (m, 3H), 7.60 (s, 1H), 8.45 (s, 1H) |
| 4 − 4 | 2.65 (s, 3H), 7.90 (s, 2H), 8.35 (s, 1H), 8.55 (s, 1H) |
| 4 − 5 | 6.90 (s, 1H), 7.65 (m, 1H), 7.80 (m, 2H), 7.90 (s, 1H), 8.70 (s, 1H) |
| 4 − 6 | 2.55 (s, 3H), 3.80 (s, 3H), 7.00~7.10 (m, 2H), 7.45~7.55 (m, 2H), 8.50 (s, 1H) |
| 4 − 7 | 2.55 (s, 3H), 3.80 (s, 3H), 6.95~7.05 (m, 1H), 7.10~7.15 (m, 2H), 7.30~7.40 (m, 1H), 8.55 (s, 1H) |
| 4 − 8 | 1.40 (s, 9H), 6.75 (d, 1H), 7.50 (s, 4H), 8.65 (s, 1H) |
| 4 − 9 | 2.60 (s, 3H), 7.30 (m, 1H), 7.50 (d, 1H), 7.60 (s, 1H), 7.70 (s, 1H), 8.50 (s, 1H) |
| 4 − 10 | 2.55 (s, 3H), 7.30~7.40 (m, 3H), 7.50 (s, 1H), 8.50 (s, 1H) |
| 4 − 11 | 2.60 (s, 3H), 7.60 (m, 1H), 7.70 (m, 2H), 7.80 (s, 1H), 8.50 (s, 1H) |
| 4 − 12 | 2.58 (s, 3H), 7.41~7.50 (m, 4H), 8.54 (s, 1H) |

Example 3 (Preparation of Formulations)

(1) Preparation of granule

Five parts by weight of Compound 40, 35 parts by weight of bentonite, 57 parts by weight of talc, 1 part by weight of Neopelex powder (trade name, available from Kao K.K.) and 2 parts by weight of sodium lignosulfate were uniformly mixed. Then, a small amount of water was added thereto to subject the mixture to mixing and kneading, and the mixture was granulated and dried to obtain granule.

(2) Preparation of wettable powder

Ten parts by weight of Compound 40, 70 parts by weight of kaolin, 18 parts by weight of white carbon, 1.5 parts by weight of Neopelex powder (trade name, available from Kao K.K.) and 0.5 part by weight of Demol (trade name, available from Kao K.K.) were mixed uniformly, and then, the mixture was pulverized to obtain a wettable powder.

(3) Preparation of emulsifiable concentrate

Ten parts by weight of Toxanone (trade name, available from Sanyo Kasei Kogyo) were added to 20 parts by weight of Compound 40 and 70 parts by weight of xylene, and the mixture was mixed uniformly and dissolved to obtain an emulsifiable concentrate.

(4) Preparation of dustable powder

Five parts by weight of Compound 40, 50 parts by weight of talc and 45 parts by weight of kaolin were uniformly mixed to obtain a dustable powder.

Example 4 (tests of effects)

(1) Antibacterial tests against respective phytopathogenic fungi

Into a culture dish made of a plastic having a diameter of 9 cm was poured a potato dextrose agar medium (PDA medium) mixed with 10,000 ppm acetone solution of Compound (1) shown in Table 1 so as to have a concentration of 20 ppm or 5 ppm whereby a agar plate culture was prepared.

Incidentally, in the column of the compounds shown in Table 7 which shows the results of antibacterial tests against respective plant bacteria mentioned below, a compound shown with netting was tested with 5 ppm, and a compound shown with not netting was tested with 20 ppm.

Antibacterial tests of mold were carried out by cutting a myceliae tuft to be tested which were previously cultured on the PDA medium with a size of about 3 mm square and it was inoculated to the above-mentioned chemical-containing PDA medium.

As for potato disease bacteria, a rye medium was used in place of a PDA medium.

After inoculating mold to a flat culture medium, growth state of the mold cultured at a temperature suitable for growth for 1 to 7 days was measured by a diameter of a myceliae tuft. It was compared with a diameter of a myceliae tuft in a chemical-free district to calculate a growth-inhibiting ratio of the myceliae tuft, and the evaluation standard with 6 steps as shown in the following Table 5 was employed.

Table 5

| Evaluation standard | Inhibiting ratio of growing bacteria (%) |
|---|---|
| 5 | Less than 100 to 95 |
| 4 | Less than 95 to 85 |
| 3 | Less than 85 to 70 |
| 2 | Less than 70 to 45 |
| 1 | Less than 45 to 10 |
| 0 | Less than 10 to 0 |

Antibacterial test of bacteria was carried out by inoculating to the above-mentioned chemical-containing PDA medium 10 μl of a cultured material which was obtained by inoculating a loopful of bacteria to be tested which were cultured previously in a PDA medium to a Wakimoto liquid medium and culturing at 25 °C for 24 hours. Then, it was cultured at 25 °C for 3 days and compared with the growth of bacteria at the chemical-free district, and the evaluation standard with 3 steps as shown in the following Table 6 was employed.

Table 6

| Evaluation standard | Growth of bacteria |
|---|---|
| 5 | Completely not admitted. |
| 2 | It can be admitted but restricted than the chemical-free district. |
| 0 | It can be admitted as in the same as the chemical-free district. |

The results of the antibacterial test investigated with regard to the respective phytopathogenic fungi are shown in Table 7.

Incidentally, in the compounds in the table, Compounds 59, 60, 65 to 74, 78 and 80 to 82 were tested with 5 ppm, and the other compounds were tested with 20 ppm.

Table 7

| Compound | P.i. | P.sp. | S.s. | V.m. | M.f. | G.c. | V.i. | G.f. | C.m. | P.o. | A.a. | B.c. | C.I. | R.sp. | R.s. | X.c. | P.s. |
|---|---|---|---|---|---|---|---|---|---|---|---|---|---|---|---|---|---|
| 1 | 3 | 2 | 5 | 2 | 5 | 3 | 3 | 1 | 2 | 2 | 2 | 4 | 4 | 5 | 5 | 0 | 5 |
| 2 | 5 | 5 | 5 | 4 | 5 | 5 | 2 | 3 | 3 | 5 | 3 | 5 | 5 | 5 | 5 | 0 | 5 |
| 3 | 5 | 5 | 5 | 5 | 5 | 5 | 5 | 3 | 4 | 5 | 5 | 5 | 5 | 5 | 5 | 2 | 5 |
| 4 | 5 | 5 | 5 | 5 | 5 | 5 | 5 | 4 | 3 | 5 | 2 | 4 | 5 | 5 | 5 | NT | 0 |
| 5 | 5 | 5 | 5 | 5 | 5 | 5 | 5 | 4 | 3 | 5 | 3 | 5 | 5 | 5 | 5 | NT | 5 |
| 6 | 5 | 5 | 5 | 5 | 5 | 5 | 5 | 5 | 2 | 5 | 3 | 5 | 5 | 5 | 5 | NT | 5 |
| 7 | 5 | 5 | 5 | 5 | 5 | 5 | 5 | 2 | 3 | 5 | 3 | 3 | 5 | 5 | 5 | NT | 5 |
| 8 | 5 | 5 | 5 | 5 | 5 | 5 | 5 | 5 | 3 | 5 | 5 | 5 | 5 | 5 | 5 | NT | 5 |
| 9 | 5 | 5 | 5 | 5 | 5 | 5 | 3 | 5 | 5 | 5 | 5 | 5 | 5 | 5 | 5 | NT | 5 |
| 10 | 5 | 5 | 5 | 5 | 5 | 5 | 5 | 5 | 3 | 5 | 4 | 4 | 5 | 5 | 5 | NT | 5 |
| 11 | 5 | 5 | 5 | 5 | 5 | 5 | 5 | 5 | 3 | 5 | 4 | 5 | 5 | 5 | 5 | NT | 5 |
| 12 | 5 | 5 | 5 | 3 | 5 | 4 | 5 | 2 | 3 | 5 | 2 | 2 | 5 | 4 | 5 | NT | 2 |
| 13 | 2 | 0 | 5 | 5 | 5 | 3 | 4 | 2 | 3 | 4 | 2 | 2 | 5 | 5 | 5 | 0 | 0 |
| 14 | 5 | 5 | 5 | 5 | 5 | 5 | 4 | 3 | 3 | 5 | 2 | 4 | 5 | 5 | 5 | 5 | 5 |
| 15 | 2 | 1 | 2 | 1 | 5 | 2 | 1 | 1 | 2 | 1 | 1 | 1 | 3 | 4 | 5 | 0 | 0 |
| 16 | 5 | 5 | 3 | 2 | 5 | 3 | 4 | 2 | 3 | 4 | 2 | 3 | 3 | 4 | 5 | 0 | 0 |
| 17 | 5 | 5 | 5 | 5 | 5 | 5 | 3 | 2 | 3 | 5 | 2 | 5 | 5 | 4 | 5 | 0 | 0 |
| 18 | 5 | 5 | 5 | 5 | 5 | 5 | 4 | 3 | 4 | 5 | 4 | 5 | 5 | 5 | 5 | 2 | 5 |

Table 7 (contd)

| Compound | P.i. | P.sp. | S.s. | V.m. | M.f. | G.c. | V.i. | G.f. | C.m. | P.o. | A.a. | B.c. | C.I. | R.sp. | R.s. | X.c. | P.s. |
|---|---|---|---|---|---|---|---|---|---|---|---|---|---|---|---|---|---|
| 19 | 3 | 1 | 0 | 1 | 4 | 1 | 1 | 1 | 1 | 1 | 0 | 1 | 3 | 4 | 5 | 0 | 0 |
| 20 | 5 | 5 | 5 | 5 | 5 | 5 | 5 | 5 | 3 | 5 | 3 | 5 | 5 | 5 | 5 | 5 | 5 |
| 21 | 5 | 5 | 5 | 5 | 5 | 5 | 5 | 5 | 3 | 5 | 5 | 5 | 5 | 5 | 5 | 5 | 5 |
| 22 | 5 | 5 | 5 | 5 | 5 | 5 | 3 | 5 | 3 | 5 | 5 | 5 | 3 | 5 | 5 | 5 | 5 |
| 23 | 5 | 5 | 3 | 2 | 5 | 3 | 4 | 2 | 3 | 5 | 2 | 1 | 3 | 3 | 5 | 0 | 0 |
| 24 | 5 | 4 | 5 | 1 | 5 | 1 | 1 | 2 | 2 | 2 | 1 | 2 | 1 | 3 | 5 | 0 | 0 |
| 25 | 5 | 5 | 5 | 5 | 5 | 5 | 3 | 4 | 3 | 5 | 3 | 5 | 5 | 5 | 5 | 5 | 5 |
| 26 | 5 | 5 | 5 | 3 | 5 | 5 | 3 | 5 | 3 | 5 | 5 | 4 | 5 | 5 | 5 | 5 | 5 |
| 27 | 5 | 5 | 5 | 1 | 5 | 2 | 1 | 1 | 2 | 5 | 2 | 1 | 1 | 2 | 4 | 0 | 0 |
| 28 | 5 | 5 | 2 | 2 | 5 | 2 | 3 | 1 | 2 | 5 | 1 | 1 | 1 | 1 | 3 | 0 | 0 |
| 29 | 5 | 5 | 5 | 5 | 5 | 5 | 3 | 5 | 3 | 5 | 4 | 5 | 5 | 5 | 5 | 5 | 5 |
| 30 | 5 | 5 | 5 | 3 | 5 | 5 | 5 | 2 | 3 | 5 | 2 | 3 | 5 | 2 | 5 | 2 | 2 |
| 31 | 5 | 5 | 5 | 1 | 5 | 2 | 2 | 2 | 2 | 2 | 2 | 1 | 1 | 5 | 5 | 5 | 5 |
| 32 | 5 | 5 | 1 | 2 | 5 | 2 | 1 | 1 | 2 | 3 | 1 | 1 | 1 | 3 | 2 | 5 | 5 |
| 33 | 5 | 5 | 5 | 5 | 5 | 5 | 3 | 2 | 2 | 5 | 2 | 1 | 3 | 5 | 5 | 5 | 5 |
| 34 | 5 | 5 | 5 | 3 | 5 | 3 | 2 | 2 | 3 | 5 | 2 | 4 | 3 | 5 | 5 | 2 | 0 |
| 35 | 3 | 3 | 4 | 2 | 5 | 2 | 1 | 1 | 2 | 2 | 1 | 1 | 2 | 3 | 2 | 2 | 0 |
| 36 | 5 | 5 | 5 | 1 | 4 | 1 | 1 | 2 | 2 | 1 | 2 | 1 | 2 | 5 | 5 | 2 | 5 |

Table 7 (contd)

| Compound | P.i. | P.sp. | S.s. | V.m. | M.f. | G.c. | V.i. | G.f. | C.m. | P.o. | A.a. | B.c. | C.l. | R.sp. | R.s. | X.c. | P.s. |
|---|---|---|---|---|---|---|---|---|---|---|---|---|---|---|---|---|---|
| 37 | 5 | 5 | 5 | 5 | 5 | 5 | 3 | 5 | 2 | 5 | 5 | 5 | 4 | 5 | 5 | 5 | 5 |
| 38 | 5 | 5 | 5 | 5 | 5 | 5 | 3 | 5 | 3 | 5 | 4 | 5 | 5 | 5 | 5 | 5 | 5 |
| 39 | 5 | 5 | 2 | 2 | 5 | 2 | 2 | 1 | 3 | 3 | 2 | 1 | 3 | 3 | 3 | 0 | 5 |
| 40 | 5 | 5 | 5 | 5 | 5 | 5 | 4 | 5 | 5 | 5 | 5 | 5 | 5 | 5 | 5 | 5 | 5 |
| 41 | 5 | 5 | 5 | 5 | 5 | 5 | 4 | 5 | 5 | 5 | 0 | 5 | 5 | 5 | 5 | 5 | 5 |
| 42 | 2 | 4 | 5 | 2 | 5 | 2 | 3 | 1 | 3 | 5 | 3 | 2 | 3 | 5 | 5 | 0 | 0 |
| 43 | 5 | 5 | 5 | 2 | 5 | 3 | 4 | 1 | 3 | 4 | 2 | 3 | 4 | 5 | 5 | 0 | 0 |
| 44 | 5 | 5 | 5 | 5 | 5 | 5 | 3 | 2 | 3 | 5 | 2 | 2 | 3 | 5 | 5 | 2 | 5 |
| 45 | 5 | 5 | 3 | 3 | 5 | 3 | 3 | 1 | 3 | 5 | 3 | 3 | 3 | 5 | 5 | 5 | 5 |
| 59 | 5 | 5 | 5 | 2 | 5 | 4 | 2 | 1 | 2 | 2 | 1 | 2 | 2 | 3 | 0 | 5 | 5 |
| 60 | 5 | 5 | 5 | 2 | 5 | 3 | 2 | 1 | 2 | 2 | 1 | 1 | 2 | 3 | 0 | 4 | 5 |
| 63 | 5 | 5 | 5 | 5 | 5 | 5 | 3 | 5 | 3 | 5 | 3 | 2 | 2 | 4 | 0 | 5 | 5 |
| 64 | 5 | 5 | 5 | 5 | 5 | 3 | 3 | 2 | 3 | 3 | 2 | 2 | 2 | 4 | 0 | 5 | 5 |
| 65 | 5 | 5 | 1 | 1 | 4 | 1 | 1 | 1 | 2 | 2 | 1 | 1 | 1 | 1 | 0 | 2 | 4 |
| 66 | 5 | 5 | 5 | 1 | 4 | 1 | 1 | 1 | 2 | 1 | 1 | 1 | 1 | 2 | 0 | 3 | 5 |
| 67 | 5 | 5 | 5 | 1 | 5 | 2 | 1 | 2 | 2 | 1 | 1 | 1 | 1 | 2 | 0 | 5 | 5 |
| 68 | 5 | 5 | 5 | 1 | 5 | 2 | 1 | 2 | 2 | 2 | 1 | 1 | 2 | 2 | 0 | 5 | 5 |
| 69 | 5 | 5 | 5 | 1 | 5 | 2 | 1 | 2 | 2 | 5 | 1 | 1 | 1 | 2 | 0 | 5 | 5 |

Table 7 (contd)

| Compound | P.i. | P.sp. | S.s. | V.m. | M.f. | G.c. | V.i. | G.f. | C.m. | P.o. | A.a. | B.c. | C.l. | R.sp. | R.s. | X.c. | P.s. |
|---|---|---|---|---|---|---|---|---|---|---|---|---|---|---|---|---|---|
| 7 0 | 5 | 5 | 5 | 1 | 5 | 2 | 1 | 2 | 2 | 5 | 1 | 1 | 1 | 3 | 0 | 5 | 5 |
| 7 1 | 5 | 5 | 5 | 1 | 5 | 2 | 1 | 1 | 1 | 1 | 1 | 1 | 1 | 2 | 0 | 3 | 5 |
| 7 2 | 5 | 5 | 5 | 1 | 5 | 2 | 1 | 2 | 2 | 2 | 1 | 1 | 1 | 2 | 0 | 5 | 5 |
| 7 3 | 5 | 5 | 5 | 1 | 5 | 2 | 1 | 2 | 2 | 5 | 1 | 1 | 1 | 2 | 0 | 4 | 5 |
| 7 4 | 5 | 5 | 5 | 2 | 5 | 3 | 1 | 2 | 3 | 5 | 1 | 2 | 2 | 3 | 0 | 4 | 5 |
| 7 5 | 5 | 5 | 5 | 5 | 5 | 5 | 5 | 5 | 5 | 5 | 2 | 5 | 5 | 5 | 0 | 5 | 5 |
| 7 6 | 5 | 5 | 1 | 1 | 5 | 2 | 3 | 1 | 2 | 2 | 1 | 1 | 1 | 1 | 0 | 3 | 5 |
| 7 7 | 5 | 5 | 1 | 1 | 1 | 4 | 1 | 1 | 1 | 1 | 1 | 1 | 1 | 2 | 0 | 3 | 5 |
| 7 8 | 5 | 5 | 1 | 2 | 1 | 5 | 3 | 1 | 2 | 1 | 1 | 1 | 2 | 2 | 0 | 3 | 5 |
| 8 0 | 5 | 5 | 5 | 1 | 5 | 2 | 1 | 1 | 2 | 1 | 1 | 2 | 2 | 3 | 0 | 3 | 5 |
| 8 1 | 5 | 5 | 5 | 1 | 4 | 2 | 1 | 1 | 2 | 1 | 1 | 1 | 1 | 2 | 0 | 4 | 5 |
| 8 2 | 5 | 5 | 1 | 0 | 1 | 1 | 2 | 0 | 0 | 0 | 0 | 1 | 0 | 2 | 0 | 0 | 1 |

Abbreviated symbols in the Tables are as mentioned below.

P.i.: *Phytophthora infestans* (Potato late blight fungi)

P.sp.: *Phytium sp.* (Phytium fungi)

S.s.: *Sclerotinia sclerotiorum* (cucumber sclerotinia rot fungi)

V.m.: *Valsa mali* (Japanese apple canker fungi)

M.f.: *Monilinia fructicola* (plum brown rot fungi)

G.c.: *Glomerella cingulata* (grape ripe rot fungi)

V.i.: *Venturia inaequalis* (apple scab fungi)

G.f.: *Gibberella fujikuroi* (rice Bakanae disease fungi)

C.m.: *Cochlioboulus miyabeanus* (rice brown spot)

P.o.: *Pyricularia oryzae* (rice blast fungi)

A.a.: *Altemaria altemata* (pearblack spot fungi)

B.c.: *Botrytis cinerea* (cucumber gray mold fungi)

C.l.: *Colletotrichum lagenarium* (cucumber anthracnose fungi)

R.sp.: *Rhizoctonia sp.* (Rhizoctonia fungi)

R.s.: *Rhizoctonia solani* (rice sheath blight fungi)

X.c.: *Xanthomonas campestris pv.citr* (citrus canker fungi)

P.s.: *Pseudomonas solanacearum* (eggplant fungil wilt fungi)

NT: not tested(not tested)

(2) Test for controlling effect on cucumber downy mildew (prevention effect)

In plastic flowerpots having a diameter of 6 cm, one cucumber (variety; Sagami Hanshiro) was grown per one flower pot, and to the young plants at 1.5 leaf stage, the chemicals obtained by diluting the wettable powders of the compounds (1) shown in Table 1 prepared as in Example 3 to 500 ppm with water containing a surfactant (0.01 %) were sprayed in an amount of 20 ml per one flowerpot, respectively.

After spraying, the cucumbers were grown in a glass greenhouse for 2 days, and then, zoosporangia of cucumber downy mildew bacteria (*Pseudoperonospora cubensis*) was prepared ($10^5$ zoosporangia/ml) from infected leaves and sprayed uniformly to the back surfaces of the plant leaves to be inoculated thereinto.

After inoculation, the cucumbers were kept in a dark place at 20 °C under moist chamber conditions for one day and night, and grown in a glass greenhouse for 5 days, and the degree of lesion of cucumber downy mildew appeared on the first leaves was examined.

Evaluation of chemicals controlling effects was effected by obtaining a controlling rate from the ratio of lesion area of the treated district based on the non-treated district, and the evaluation standard with 6 steps as shown in the following Table 8 was employed.

Table 8

| Evaluation standard | Inhibiting ratio of growing bacteria (%) |
|---|---|
| 5 | 100 |
| 4 | Less than 100 to 95 |
| 3 | Less than 95 to 80 |
| 2 | Less than 80 to 60 |
| 1 | Less than 60 to 30 |
| 0 | Less than 30 to 0 |

The results of the evaluation were shown in Table 9.

Table 9 Test of controlling effect on cucumber downy mildew

| Compound | Effect | Compound | Effect |
|---|---|---|---|
| 6 | 5 | 26 | 4 |
| 13 | 4 | 31 | 5 |
| 14 | 4 | 32 | 4 |
| 19 | 4 | 37 | 4 |
| 20 | 4 | 38 | 4 |
| 21 | 4 | 40 | 5 |
| 22 | 4 | | |

(3) Test of controlling effect on cucumber gray mold (Paper disc test)

Paper moisturized by distilled water was spread all over in a plastic apparatus (35 cm x 25 cm) and seed leaves of cucumber (variety: Sagami Hanshiro) was placed thereon, then, a suspension of lesion of cucumber gray mold (a suspension of $10^5$ spores/ml prepared by a solution of 5% of sugar and 1% yeast extract) was dropped on the seed leaves in an amount of 50 μl, and a paper disc was further placed thereon.
Chemicals obtained by diluting the wettable powders of the compounds (1) shown in Table 1 prepared as in Example 3 to 500 ppm with water containing a surfactant (0.05 %) were immediately dropped on the paper disc in an amount of 90 μl, respectively.
After putting a lid on the apparatus and sealing it with a vinyl tape, the seed leaves were stored at 20°C for 4 days, and then, the degree of lesion of cucumber gray mold appeared on the seed leaves was examined.
Evaluation of the effects of the chemicals was shown in the following Table 10 with the 6 rank evaluation standard obtained by the same manner as in the above-mentioned (2).

Table 10 Test of controlling effect on cucumber gray mold

| Compound | Effects | Compound | Effects |
|---|---|---|---|
| 1 | 5 | 12 | 5 |
| 2 | 5 | 16 | 4 |
| 4 | 4 | 17 | 4 |
| 5 | 5 | 21 | 5 |
| 6 | 5 | 22 | 5 |
| 7 | 5 | 25 | 5 |
| 8 | 5 | 30 | 4 |
| 9 | 5 | 40 | 5 |
| 10 | 5 | 45 | 5 |
| 11 | 5 | | |

(4) Test of controlling effect on rice blast (prevention effect)

In plastic flowerpots having a diameter of 6 cm, 10 rice seedlings (variety: Nihonbare) were grown per one flowerpot, and to the young plants at 2.5 leaf stage, chemicals of respective wettable powders of the compounds (1) prepared as in Example 3 shown in Table 3 obtained by diluting with water containing a surfactant (0.05 %) to 500 ppm was sprayed in an amount of 20 ml per one flowerpot, respectively.
On the next day after spraying, a suspension of conidiospores of rice blast prepared from infected leaves (3 x $10^5$ spores/ml) was sprayed uniformly to the plant leaves to be inoculated thereto.
After inoculation, the rice seedlings were grown in a moist chamber at 25°C for 4 days (the first three days are under dark place, and the remaining one day is under a light), and the degree of lesion of rice blast appeared on the leaves was examined.
Evaluation of the effects of the chemicals was shown in the following Table 11 with the 6 rank protective values obtained by the same manner as in the above-mentioned (2).

Table 11 Test of controlling effect on rice blast (prevention effect)

| Compound | Effects | Compound | Effects |
|---|---|---|---|
| 1 | 5 | 21 | 5 |
| 2 | 5 | 22 | 4 |
| 3 | 4 | 24 | 4 |
| 5 | 5 | 27 | 4 |
| 6 | 5 | 29 | 4 |
| 8 | 5 | 30 | 4 |
| 9 | 5 | 33 | 5 |
| 10 | 4 | 34 | 5 |
| 11 | 5 | 35 | 4 |
| 12 | 4 | 37 | 4 |
| 13 | 5 | 39 | 4 |
| 14 | 5 | 40 | 5 |
| 15 | 5 | 42 | 5 |
| 16 | 4 | 43 | 4 |
| 17 | 4 | 44 | 5 |
| 18 | 4 | 45 | 5 |
| 20 | 4 | | |

(5) Test of controlling effect on wheat brown rust (prevention effect)

In plastic flowerpots having a diameter of 6 cm, 10 wheat seedlings (variety: Kobushi Komugi) were grown per one flowerpot, and to the young plants at 1.5 leaf stage, wettable powders of the compounds (1) shown in Table 1 prepared as in Example 3 were diluted with water containing a surfactant (0.05 %) to 500 ppm and these respective solutions were sprayed in an amount of 20 ml per one flowerpot, respectively.
On the next day after spraying, a suspension of spores of wheat brown rust (5 x $10^5$ spores/ml) was sprayed uniformly to the plants to be inoculated thereto.
After inoculation, the wheat seedlings were stored at a dark place at 20 °C under moist chamber state over a day and night, and grown in a glass greenhouse for 9 days, and the degree of lesion of wheat brown rust appeared on the first leaves was examined.
Evaluation of the effects of the chemicals was shown in the following Table 12 with the 6 rank protective values obtained by the same manner as in the above-mentioned (2).

Table 12 Test of controlling effects on wheat brown rust (prevention effect)

| Compound | Effects | Compound | Effects |
|---|---|---|---|
| 1 | 4 | 25 | 4 |
| 2 | 4 | 26 | 4 |
| 3 | 5 | 33 | 4 |
| 13 | 4 | 34 | 4 |
| 14 | 5 | 35 | 4 |
| 15 | 4 | 40 | 5 |
| 17 | 5 | 41 | 4 |
| 18 | 4 | 42 | 4 |
| 20 | 4 | 43 | 3 |
| 21 | 4 | 44 | 3 |
| 22 | 4 | 45 | 3 |

(6) Test of controlling effect on rice "Bakanae" disease

Unhulled rices infected by rice "Bakanae" disease obtained by inoculating a suspension of rice "Bakanae" disease (*Gibberella fujikuroi*) spores to rice (variety: Waito C) at the flowering time were provided as a sample to be tested.

The above-mentioned infected unhulled rices were dipped in a chemical solution obtained by diluting respective wettable powder of the compound (1) shown in Table 1 prepared as in Example 3 with water containing a surfactant (0.05 %) to 1000 ppm at 20 °C for 24 hours. These unhulled rices were air dried and further dipped in the chemical solution at 25 °C for 2 days, and they were sowed in a plastic pot with a diameter of 6.5 cm.

Thereafter, they were controlled in a glass greenhouse, and after 3 weeks from sowing, diseased seedlings were examined as shown below and the protective value was calculated.

$$\text{Proportion of diseased seedlings (\%)} = \frac{\text{Number of diseased seedlings}}{\text{Number of seedlings examined}} \times 100$$

$$\text{Protective value (\%)} = \left(1 - \frac{\text{Proportion of diseased seedlings at the treated district}}{\text{Proportion of diseased seedlings at the non-treated district}}\right) \times 100$$

The results are shown in Table 13 with the investigation about the presence or absence of chemical damage.

Table 13 Test of controlling effects on rice "Bakanae" disease

| Compound | Protective value (%) | Chemical damage |
|---|---|---|
| 10 | 88 | None |
| 20 | 94 | None |
| 29 | 90 | None |
| fludioxonil | 88 | None |
| Non-treated | 0 | — |

(7) Test of controlling effects on rice bacterial grain rot

A suspension of rice bacterial grain rot (*Pseudomonas glumae*) obtained by culturing in a Wakimoto livid medium by shaking at 25 °C for 24 hours (about 1.5 x $10^9$ /ml) was inoculated to dried unhulled rices (variety: Tangin Bozu), they were allowed to stand at 30 °C for 4 hours and air-dried for one hour, and then inoculated.

The inoculated unhulled rices were dipped in a chemical solution obtained by diluting respective wettable powders of the compounds (1) shown in Table 1 with water containing a surfactant (0.05 %) prepared as in Example 3 to 1000 ppm at 20 °C for 24 hours. These unhulled rices were air-dried over one hour, sowed in a plastic pot having a diameter of 6.5 cm and germinated at 30 °C under humid conditions.

Thereafter, they were controlled in a glass green house, and after two weeks from sowing, diseased seedlings were examined and the protective value was calculated.

The results are shown in Table 14 with the investigation about the presence or absence of chemical damage.

Table 14 Test of controlling effects on rice bacterial grain rot

| Compound | Protective value (%) | Chemical damage |
|---|---|---|
| 8 | 93 | None |
| 10 | 100 | None |
| 20 | 86 | None |
| Starner | 70 | None |
| Non-treated | 0 | — |

Utilizability in industry

The novel pyrimidine compound of the present invention has excellent fungicidal effect for agricultural and horticultural use and available as a fungicide for agricultural and horticultural use.

## Claims

1. A pyrimidine compound represented by the following formula (1):

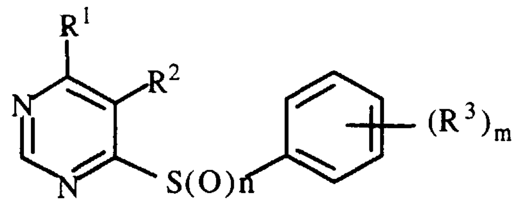

(1)

wherein $R^1$ represents an alkyl group having 1 to 4 carbon atoms, a halogen atom or a hydrogen atom; $R^2$ represents a halogen atom, an alkyl group having 1 to 4 carbon atoms or a hydrogen atom; $R^3$ represents an alkyl group having 1 to 8 carbon atoms, a haloalkyl group having 1 to 4 carbon atoms, a halogen atom, a nitro group, an alkoxy group having 1 to 4 carbon atoms, a substituted or unsubstituted benzoylamino group, a substituted or unsubstituted phenoxy group, an alkenyloxy group having 2 to 5 carbon atoms, an alkynyloxy group having 2 to 5 carbon atoms, a cycloalkylcarbonylamino group having 4 to 9 carbon atoms, a haloalkylcarbonyl amino group having 2 to 5 carbon atoms, a cyano group, a formyl group, an acyl group having 2 to 5 carbon atoms, a hydrogen atom or an alkylsulfonyl group having 1 to 4 carbon atoms; m represents an integer of 0 to 5; and n represents 1 or 2.

2. The pyrimidine compound according to Claim 1, wherein $R^1$ is a methyl group, an ethyl group, a chlorine atom or a hydrogen atom.

3. The pyrimidine compound according to Claim 1, wherein $R^2$ is a methyl group, an ethyl group, a chlorine atom or a hydrogen atom.

4. The pyrimidine compound according to Claim 1, wherein $R^3$ is $CH_3$, $C_2H_5$, n-$C_3H_7$, i-$C_3H_7$, n-$C_4H_9$, s-$C_4H_9$, t-$C_4H_9$, $CF_3$, a chlorine atom, a bromine atom, a fluorine atom, a nitro group, $OCH_3$, a fluorine atom-substituted or unsubstituted benzoylamino group, a nitro group-substituted or unsubstituted phenoxy group, $OCH_2CH{=}CH_2$, $OCH_2C{\equiv}CH$ a cyclopropylcarbonylamino group, a chloromethylcarbonylamino group, a cyano group, a formyl group, an acetyl group, a hydrogen atom or a methylsulfonyl group.

5. The pyrimidine compound according to Claim 1, wherein $R^1$ is a methyl group, an ethyl group, a chlorine atom or a hydrogen atom; $R^2$ is a methyl group, an ethyl group, a chlorine atom or a hydrogen atom; $R^3$ is $CH_3$, $C_2H_5$, n-$C_3H_7$, i-$C_3H_7$, n-$C_4H_9$, s-$C_4H_9$, t-$C_4H_9$, $CF_3$, a chlorine atom, a bromine atom, a fluorine atom, a nitro group, $OCH_3$, a fluorine atom-substituted or unsubstituted benzoylamino group, a nitro group-substituted or unsubstituted phenoxy group, $OCH_2CH{=}CH_2$, $OCH_2C{\equiv}CH$, a cyclopropylcarbonylamino group, a chloromethylcarbonylamino group, a cyano group, a formyl group, an acetyl group, a hydrogen atom or a methylsulfonyl group; m represents an integer of 0 to 2; and n is 1 or 2.

6. A compound represented by the following formula (4):

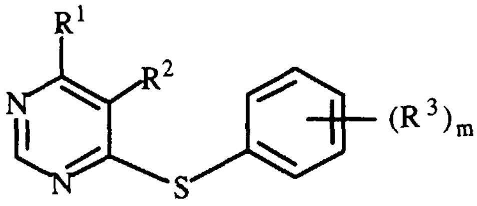

(4)

wherein $R^1$ to $R^3$ and m have the same meanings as defined in Claim 1.

**7.** The compound according to Claim 6, wherein $R^1$ is a methyl group, an ethyl group, a chlorine atom or a hydrogen atom; $R^2$ is a methyl group, an ethyl group, a chlorine atom or a hydrogen atom; $R^3$ is $CH_3$, $C_2H_5$, n-$C_3H_7$, i-$C_3H_7$, n-$C_4H_9$, s-$C_4H_9$, t-$C_4H_9$, $CF_3$, a chlorine atom, a bromine atom, a fluorine atom, a nitro group, $OCH_3$, a fluorine atom-substituted or unsubstituted benzoylamino group, a nitro group-substituted or unsubstituted phenoxy group, $OCH_2CH{=}CH_2$, $OCH_2C{\equiv}CH$, a cyclopropylcarbonylamino group, a chloromethylcarbonylamino group, a cyano group, a formyl group, an acetyl group, a hydrogen atom or a methylsulfonyl group; and m represents an integer of 0 to 2.

**8.** A process for producing a pyrimidine compound represented by the formula (1) according to Claim 1 which comprises oxidizing a compound of the following formula (4):

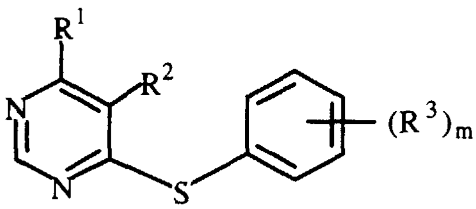

(4)

wherein $R^1$ to $R^3$ and m have the same meanings as defined in Claim 1,

which is obtained by reacting a compound represented by the following formula (2):

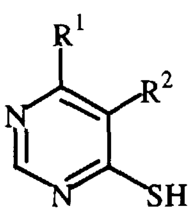

(2)

wherein $R^1$ and $R^2$ have the same meanings as defined in Claim 1,

and a compound represented by the following formula (3):

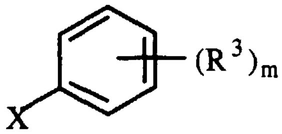

(3)

wherein $R^3$ has the same meaning as defined in Claim 1; and X represents a halogen atom.

**9.** A process for preparing the pyrimidine compound represented by the formula (1) according to Claim 1 which comprises oxidizing a compound of the following formula (4):

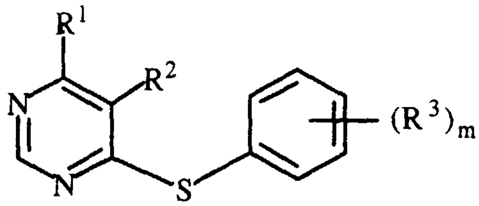

(4)

wherein $R^1$ to $R^3$ and m have the same meanings as defined in Claim 1,

which is obtained by reacting a compound represented by the following formula (5):

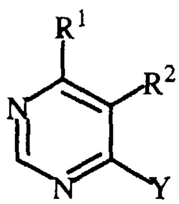

(5)

wherein $R^1$ and $R^2$ have the same meanings as defined in Claim 1. Y represents a halogen atom,

and a compound represented by the following formula (6):

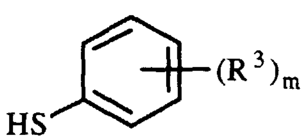

(6)

wherein $R^3$ and m have the same meanings as defined in Claim

1.

**10.** An agricultural and horticultural fungicide containing the pyrimidine compound represented by the formula (1) according to Claim 1 as an effective ingredient.

## INTERNATIONAL SEARCH REPORT

International application No.
PCT/JP97/00969

A. CLASSIFICATION OF SUBJECT MATTER

Int. $Cl^6$ C07D239/38, A01N43/54

According to International Patent Classification (IPC) or to both national classification and IPC

B. FIELDS SEARCHED

Minimum documentation searched (classification system followed by classification symbols)

Int. $Cl^6$ C07D239/38, A01N43/54

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| A | JP, 5-255311, A (CIBA-Geigy AG.), October 5, 1993 (05. 10. 93), Document as a whole & EP, 549943, A | 1 - 10 |
| A | JP, 6-9626, A (CIBA-Geigy AG.), January 18, 1994 (18. 01. 94), Document as a whole & EP, 549532, A | 1 - 10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| May 27, 1997 (27. 05. 97) | June 3, 1997 (03. 06. 97) |
| Name and mailing address of the ISA/ Japanese Patent Office | Authorized officer |
| Facsimile No. | Telephone No. |

Form PCT/ISA/210 (second sheet) (July 1992)